Europäisches Patentamt

(19) European Patent Office

Office européen des brevets

(11) Numéro de publication: **0 183 628**
**B1**

(12) **FASCICULE DE BREVET EUROPEEN**

(45) Date de publication du fascicule du brevet:
26.08.87

(51) Int. Cl.⁴: **C 07 C 46/00, C 07 C 50/16**

(21) Numéro de dépôt: **85420189.4**

(22) Date de dépôt: **22.10.85**

(54) Procédé de préparation d'amyl-2-anthraquinone à partir d'amyl-benzène et d'anhydride phtalique.

(30) Priorité: **31.10.84 FR 8416828**

(43) Date de publication de la demande:
**04.06.86 Bulletin 86/23**

(45) Mention de la délivrance du brevet:
**26.08.87 Bulletin 87/35**

(84) Etats contractants désignés:
**AT BE CH DE FR GB IT LI NL SE**

(56) Documents cités:
**EP - A - 0 055 951**
**EP - A - 0 121 466**
**US - A - 4 035 396**

(73) Titulaire: **ATOCHEM, 4 & 8, Cours Michelet La Défense 10, F-92800 Puteaux (FR)**

(72) Inventeur: **Devic, Michel, 27 Chemin des Fonds, F-69110 Ste Foy Les Lyon (FR)**

## Description

La présente invention concerne un procédé de préparation d'amyl-2-anthraquinone à partir d'amyl-benzène et d'anhydride phtalique.

Elle concerne plus particulièrement un procédé de fabrication d'amyl-2 anthraquinone à teneur élevée en isomère tert.

L'amyl-2 anthraquinone est utilisée dans l'industrie en particulier dans la synthèse du peroxyde d'hydrogène par le procédé dit à l'anthraquinone dans lequel elle est préférée à l'éthyl-2-anthraquinone ou la tert.butyl-2-anthraquinone.

Dans cette application, des mélanges riches en isomère tert.amyl-2-anthraquinone sont particulièrement intéressants à mettre en œuvre parce que permettant une vitesse de réaction élevée et une forte productivité.

Un tel avantage est par exemple signalé dans le brevet français publié sous le numéro 2 044 590 ou dans le brevet des Etats-Unis d'Amérique n° 3 041 143.

L'intérêt est donc évident pour l'industrie de disposer d'un procédé rendant possible l'obtention d'amyl-2-anthraquinone contenant une proportion aussi élevée que possible, de préférence supérieure à 80% d'isomère tert.

Le procédé de préparation de l'amyl-2-anthraquinone par condensation du tert.amylbenzène avec l'anhydride phtalique en présence de AlCl₃ puis cyclisation de l'acide amyl-orthobenzoylbenzoïque en présence d'acide sulfurique ou d'oléum, ne permet pas d'atteindre un tel résultat.

Ceci est particulièrement mis en évidence dans le brevet français publié sous le numéro 2 351 083: lorsque le tert.amylbenzène et l'anhydride phtalique réagissent en présence d'un acide de Lewis, une portion du radical tert-amyle est isomérisée et il est impossible de dépasser une proportion d'isomère tert. de 55%.

Ce taux, selon la demande de brevet japonais publiée sous le numéro 73/75558, atteint cependant 70% environ lorsqu'on opère en présence d'un fort excès d'anhydride phtalique.

Cette valeur représente un maximum selon le brevet français n° 2 044 590 déjà cité.

Ce dernier brevet propose bien une voie d'accès à la tert.amyl-2-anthraquinone mais elle présente des inconvénients majeurs tant sur le plan économique que sur le plan de la sécurité, compte tenu du nombre et de la nature des étapes qu'elle comprend: formation qui en dérive et finalement condensation de cet organo-magnésien avec l'anhydride phtalique.

Dans le brevet français n° 2 351 083 est proposée une amélioration du procédé classique au chlorure d'aluminium due à l'injection, dans le milieu de réaction, d'un gaz inerte ou à la réduction de la pression au-dessous de la pression atmosphérique à laquelle est habituellement conduite la réaction.

Si, selon ce brevet, des taux d'isomère tert. supérieurs à 80% apparaissent possibles, ce n'est qu'au prix de conditions opératoires ne répondant pas aux exigences modernes de l'économie d'un procédé anthraquinone: durée de réaction très longue et/ou très fort excès d'anhydride phtalique et surtout de chlorure d'aluminium.

Ainsi, quand on considère les techniques mettant en jeu le chlorure d'aluminium, il apparaît que le taux d'isomère tert. est soit limité à 70% dans ces conditions de procédé compatibles avec l'économie industrielle, soit exceptionnellement supérieur à 80% dans des conditions de procédé ne répondant pas aux exigences d'une telle économie.

Le mode de synthèse d'anthraquinones en phase liquide homogène en présence d'un catalyseur constitué d'un mélange de HF et de BF₃, tel que décrit dans le brevet européen publié sous le numéro 0055951, bien que constituant un progrès déterminant dans l'économie d'une telle synthèse, ne permet pas une sélectivité encore suffisante en isomère désiré, comme il sera montré dans les exemples.

Le but de la demanderesse a été de conserver les avantages économiques qui caractérisent la synthèse d'anthraquinones en présence de catalyseurs HF-BF₃ par rapport aux procédés du type au chlorure d'aluminium, dans un procédé qui permette de minimiser l'isomérisation.

Le procédé selon l'invention, qui consiste à préparer l'amyl-2-anthraquinone par réaction de l'anhydride phtalique avec le tert-amylbenzène en présence d'un catalyseur constitué d'un mélange de HF et de BF₃ puis transformation de l'acide amyl-orthobenzoylbenzoïque obtenu en amyl-2-anthraquinone suivant des procédés connus en soi, est caractérisé en ce que la réaction de l'anhydride phtalique avec le tert.amylbenzène est effectuée à une température comprise entre −60° C et −20° C dans un milieu de réaction liquide biphasique formé grâce à la présence d'un solvant du tert. amylbenzène, inerte et non miscible avec le mélange de HF et de BF₃ dans les conditions de la réaction.

Pour constituer le milieu de réaction, l'ordre d'introduction de ses constituants n'a pas une influence déterminante sur le résultat mais il est normalement conseillé d'introduire le tert.amylbenzène en dernier et de préférence en solution dans le solvant.

Parmi les solvants, employés seuls ou en mélange, qui conviennent à la réalisation de l'invention, on peut citer: le $CO_2$ liquide, les hydrocarbures saturés linéaires ou cycliques, ramifiés ou non, comportant jusqu'à 8 atomes de carbone, dans la formule desquels un ou plusieurs atomes d'hydrogène peuvent être substitués par un ou plusieurs atomes de chlore et/ou de fluor, liquides dans les conditions de la réaction.

Les solvants préférés sont le propane et le chlorure de méthylène à cause de leur coût et des résultats qu'ils procurent.

La quantité de solvant mis en œuvre dépend des conditions choisies pour la réaction mais doit être nécessairement suffisante pour que le milieu de réaction comporte deux phases liquides distinctes.

Généralement cette quantité de solvant est comprise, en volume, entre 2 et 15 parties, de préférence entre 3 et 10 parties par partie de tert. amyl-benzène.

Le rapport molaire HF/BF$_3$ est de préférence choisi entre 0,2 et 1,5, de préférence au plus égal à 1 et le rapport molaire tert.amylbenzène/anhydride phtalique compris entre 0,5 et 1,5.

Dans les conditions préférées pour les autres paramètres de la réaction, celle-ci se déroule particulièrement bien lorsqu'elle est effectuée à une température comprise entre −40° C et −30° C.

La durée de la réaction est comprise entre 5 et 60 minutes selon la température.

Selon la température encore, la réaction a lieu sous une pression généralement comprise entre 5 et 40 bar environ.

En fin de réaction la plus grande partie du catalyseur peut être récupérée par évaporation sous pression réduite à une température n'excédant pas 20° C environ, de préférence à la température à laquelle a lieu la réaction.

Le catalyseur peut être encore avantageusement récupéré en opérant selon le procédé décrit dans la demande de brevet français publiée sous le numéro 2 532 303, qui consiste à soumettre le mélange obtenu en fin de réaction à l'action d'un solvant inerte, qui peut être le solvant mis en œuvre dans la réaction, dans une colonne à distiller, à une température de 20° C au moins.

L'acide amyl-orthobenzoylbenzoïque peut être purifié par exemple par dissolution dans une solution aqueuse d'hydroxyde de sodium suivie d'une précipitation à l'aide d'un acide minéral.

L'amyl-2-anthraquinone est finalement obtenu à partir de l'acide amyl-orthobenzoylbenzoïque par tout moyen de cyclisation connu de l'homme du métier, par exemple par chauffage en présence d'acide sulfurique concentré ou d'oléum.

Les exemples 1 à 3 suivants illustrent la présente invention. Ils sont donnés à titre indicatif mais non limitatif.

L'exemple 4 est donné à titre de comparaison.

Dans les exemples ci-dessous, le terme tert. amylbenzène désigne de l'amyl-benzène renfermant 98% d'isomère tert. et séché sur tamis moléculaire 4Å.

*Exemple 1*

10 ml de chlorure de méthylène CH$_2$Cl$_2$, 24,5 g d'anhydride phtalique sont chargés dans un autoclave en acier inoxydable muni d'un système d'agitation.

Après refroidissement du mélange des composés ci-dessus à −40° C sous agitation, sont introduits à cette température 16,6 g d'HF anhydre et 56,12 g de BF$_3$ puis, en 40 minutes la solution de 24,46 g de tert.amylbenzène dans 112 ml de CH$_2$Cl$_2$.

L'introduction terminée, la réaction est conduite durant 30 minutes à −40° C. L'autoclave est ensuite mis à pression atmosphérique à cette même température et aussitôt après une solution aqueuse d'hydroxyde de sodium à 30% en poids est ajoutée jusqu'à pH alcalin.

Le mélange est porté à ébullition pour éliminer le CH$_2$Cl$_2$ et le tert.amylbenzène qui n'a pas réagi, avant d'être filtré. Le filtrat est additionné d'acide chlorhydrique concentré jusqu'à pH acide pour précipiter l'acide amyl-benzoylbenzoïque.

Après filtration du produit précipité, lavage sur filtre par de l'eau pour éliminer l'anhydride phtalique, séchage à l'étuve sous vide du produit récupéré sur filtre, 29,15 g d'un précipité blanc cristallin sont finalement obtenus qui s'avèrent être de l'acide amyl-benzoylbenzoïque de pureté supérieure à 99%.

L'analyse par résonance magnétique nucléaire (RMN) révèle une teneur de 89% en isomère tert. dans l'acide amyl-orthobenzoylbenzoïque ainsi fabriqué.

Cet acide, cyclisé à l'aide d'un oléum à 2%, conduit à de l'amyl-2-anthraquinone contenant plus de 92% d'isomère tert.

*Exemple 2*

24,5 g d'anhydride phtalique, 24,5 g de tert. amylbenzène et 77 ml de n-hexane sont introduits dans l'autoclave de l'exemple 1 et le mélange est refroidi sous agitation à −40° C.

16,6 g d'HF et 57 g de BF$_3$ sont alors introduits dans l'autoclave à cette température.

Après 1 h de réaction à 40° C sous agitation le processus décrit dans l'exemple 1 est répété jusqu'à l'étape de séchage à l'étuve à vide comprise.

18,73 g d'acide amyl-orthobenzoylbenzoïque sont obtenus qui renferment 95% d'isomère tert.

*Exemple 3*

Après introduction dans l'autoclave des mêmes quantités d'anhydride phtalique et de tert.amylbenzène que dans l'exemple 2, le mélange est refroidi au moyen d'azote liquide pour pouvoir introduire 67,2 g de propane, 16,6 g de HF et 57 g de BF$_3$.

La température étant portée à −40° C, la réaction est effectuée à cette température durant 30 minutes sous agitation.

En procédant ensuite comme dans l'exemple 2, on obtient 28,84 g d'acide amyl-orthobenzoylbenzoïque titrant 87,5% d'isomère tert.

*Exemple 4* (comparatif)

En procédant comme dans l'exemple 1, mais sans CH$_2$Cl$_2$, on obtient, après l'étape de séchage à l'étuve à vide, 39 g d'un produit titrant 99% d'acide amyl-orthobenzoylbenzoïque. Cet acide ne renferme par contre que 25% d'isomère tert.

**Revendications**

1. Procédé de fabrication d'amyl-2-anthraquinone à teneur élevée en isomère tert. par réaction de l'anhydride phtalique avec le tert.amylbenzène en présence d'un catalyseur constitué par un mélange d'acide fluorhydrique et de trifluorure de bore puis transformation de l'acide amyl-orthobenzoylbenzoïque obtenu en amyl-2-anthraquinone suivant des procédés connus en soi, caractérisé en ce que la réaction de l'anhydride phtalique avec le tert.amylbenzène est effectuée à une température comprise entre −60° C et −20° C dans un milieu de réaction liquide biphasique formé grâce à la présence d'un solvant du tert.amylbenzène inerte et non miscible avec le mélange d'acide fluorhydrique et de trifluorure de bore dans les conditions de la réaction.

2. Procédé selon la revendication 1, caractérisé en ce que le solvant mis en œuvre est choisi parmi les hydrocarbures saturés linéaires ou cycliques, ramifiés ou non comportant jusqu'à 8 atomes de carbone.

3. Procédé selon la revendication 2, caractérisé en ce que le solvant est le propane.

4. Procédé selon la revendication 2, caractérisé en ce que, dans la formule du solvant, un ou plusieurs atomes d'hydrogène portés par les atomes de carbone sont substitués par un ou plusieurs atomes de chlore et/ou de fluor.

5. Procédé selon la revendication 4, caractérisé en ce que le solvant est le chlorure de méthylène.

6. Procédé selon la revendication 1, caractérisé en ce que le solvant est le dioxyde de carbone liquide.

7. Procédé selon l'une quelconque des revendications 1 à 6, caractérisé en ce que la quantité de solvant est comprise, en volume, entre 2 et 15 parties de tert.amylbenzène.

8. Procédé selon la revendication 7, caractérisé en ce que la quantité de solvant est comprise entre 3 et 10 parties par partie de tert.amylbenzène.

9. Procédé selon l'une quelconque des revendications 1 à 8, caractérisé en ce que la quantité de fluorure de bore est supérieure à 3 moles par mole d'anhydride phtalique.

10. Procédé selon l'une quelconque des revendications 1 à 9, caractérisé en ce que la quantité d'acide fluorhydrique est supérieure à 2 moles par mole d'anhydride phtalique.

11. Procédé selon l'une quelconque des revendications 1 à 10, caractérisé en ce que le rapport molaire $HF/BF_3$ est compris entre 0,2 et 1,5.

12. Procédé selon la revendication 11, caractérisé en ce que le rapport molaire $HF/BF_3$ est au plus égal à 1.

13. Procédé selon l'une quelconque des revendications 1 à 12, caractérisé en ce que le rapport molaire tert.amylbenzène/anhydride phtalique est compris entre 0,5 et 1,5.

## Claims

1. Process for manufacturing 2-amylanthraquinone having a high content of the tert-isomer, by reaction of phthalic anhydride with tert-amylbenzene in the presence of a catalyst consisting of a mixture of hydrofluoric acid and boron trifluoride, followed by conversion of the amyl-ortho-benzoylbenzoic acid obtained to 2-amylanthraquinone according to processes which are known per se, characterized in that the reaction of phthalic anhydride with tert-amylbenzene is performed at a temperature of between −60° C and −20° C in a two-phase liquid reaction medium formed as a result of the presence of a solvent for tert.amylbenzene which is inert and immiscible with the mixture of hydrofluoric acid and boron trifluoride under the conditions of the reaction.

2. Process according to Claim 1, characterized in that the solvent employed is chosen from linear or cyclic, branched or unbranched saturated hydrocarbons containing up to 8 carbon atoms.

3. Process according to Claim 2, characterized in that the solvent is propane.

4. Process according to Claim 2, characterized in that, in the formula of the solvent, one or more hydrogen atoms borne by the carbon atoms are substituted with one or more chlorine and/or fluorine atoms.

5. Process according to Claim 4, characterized in that the solvent is methylene chloride.

6. Process according to Claim 1, characterized in that the solvent is liquid carbon dioxide.

7. Process according to any one of Claims 1 to 6, characterized in that the amount of solvent is between, by volume, 2 and 15 parts of tert-amylbenzene.

8. Process according to Claim 7, characterized in that the amount of solvent is between 3 and 10 parts per part of tert-amylbenzene.

9. Process according to any one of Claims 1 to 8, characterized in that the amount of boron fluoride is greater than 3 moles per mole of phthalic anhydride.

10. Process according to any one of Claims 1 to 9, characterized in that the amount of hydrofluoric acid is greater than 2 moles per mole of phthalic anhydride.

11. Process according to any one of Claims 1 to 10, characterized in that the mole ratio $HF/BF_3$ is between 0.2 and 1.5.

12. Process according to Claim 11, characterized in that the mole ratio $HF/BF_3$ is at most equal to 1.

13. Process according to any one of Claims 1 to 12, characterized in that the mole ratio tert-amylbenzene/phthalic anhydride is between 0.5 and 1.5.

## Patentansprüche

1. Verfahren zur Herstellung von 2-Amyl-Anthrachinon mit hohem Anteil an tertiärem Isomer durch Reaktion von Phtalsäureanhydrid mit tert.-Amylbenzol in Gegenwart eines Katalysators bestehend aus einer Mischung aus Fluorwasserstoffsäure und Bortrifluorid und anschliessende Umwandlung der erhaltenen Amyl-ortho-Benzoyl-Benzoesäure in 2-Amyl-Anthrachinon in an sich bekannter Weise, dadurch gekennzeichnet, dass die Reaktion des Phtalsäureanhydrids mit dem Tert.-Amylbenzol bei einer Temperatur zwischen −60° C und −20° C in einem flüssigen zweiphasigen Reaktionsmedium ausgeführt wird, das gebildet wird durch Anwesenheit eines inerten und bei den Reaktionsbedingungen mit dem Gemisch aus Fluorwasserstoffsäure und Bortrifluorid nicht mischbaren Lösungsmittel für das tert.-Amylbenzol.

2. Verfahren nach Anspruch 1, dadurch gekennzeichnet, dass das verwendete Lösungsmittel ausgewählt wird aus den linearen oder zyklischen gesättigten, verzweigten oder nicht verzweigten Kohlenwasserstoffen mit bis zu 8 Kohlenstoffatomen.

3. Verfahren nach Anspruch 2, dadurch gekennzeichnet, dass das Solvens Propan ist.

4. Verfahren nach Anspruch 2, dadurch gekennzeichnet, dass in der Formel des Lösungsmittels ein oder mehrere mit Kohlenstoffatomen verbundene Wasserstoffatome durch ein oder mehrere Chlor- und/oder Fluoratome substituiert sind.

5. Verfahren nach Anspruch 4, dadurch gekennzeichnet, dass das Lösungsmittel Methylenchlorid ist.

6. Verfahren nach Anspruch 1, dadurch gekennzeichnet, dass das Lösungsmittel flüssiges Kohlendioxyd ist.

7. Verfahren nach einem der Ansprüche 1 bis 6, dadurch gekennzeichnet, dass die Menge des Lösungsmittels bezogen auf das Volumen zwischen 2 und 15 Teilen des tert.-Amylbenzols ausmacht.

8. Verfahren nach Anspruch 7, dadurch gekennzeichnet, dass die Menge des Lösungsmittels 3 bis 10 Teilen je Teil tert.-Amylbenzol entspricht.

9. Verfahren nach einem der Ansprüche 1 bis 8, dadurch gekennzeichnet, dass die Menge des Borfluorids mehr als 3 Mol pro Mol Phtalsäureanhydrid beträgt.

10. Verfahren nach einem der Ansprüche 1 bis 9, dadurch gekennzeichnet, dass die Menge der Fluorwasserstoffsäure mehr als 2 Mol pro Mol Stahlsäureanhydrid beträgt.

11. Verfahren nach einem der Ansprüche 1 bis 10, dadurch gekennzeichnet, dass das molare Verhältnis $HF/BF_3$ zwischen 0,2 und 1,5 liegt.

12. Verfahren nach Anspruch 11, dadurch gekennzeichnet, dass das molare Verhältnis $HF/BF_3$ bei ungefähr 1 liegt.

13. Verfahren nach einem der Ansprüche 1 bis 12, dadurch gekennzeichnet, dass das molare Verhältnis tert.-Amylbenzol/Phtalsäureanhydrid zwischen 0,5 und 1,5 liegt.